# EUROPEAN PATENT APPLICATION

(11) **EP 3 267 378 A1**
(43) Date of publication of application: **10.01.2018**
(21) Application number: 16178474.9
(22) Date of filing: 07.07.2016
(51) Int. Cl.: G06Q 10/08, G06Q 50/22, G06Q 10/00

(54) **DISTRIBUTED SYSTEM FOR RESOURCE MANAGEMENT FOR HAND HYGIENE UNITS**

(71) Applicant: Smixin SA, 2503 Bienne (CH)
(72) Inventor: Crottet, Denis, 1564 Domdidier (CH); Deckers, Jean Michel, 278397 Singapore (SG); Stutzer, Diego, 2503 Biel/Bienne (CH); Vaucher, Vincent, 2605 Sonceboz-Sombeval (CH); Chapelat, Carole, 2502 Biel/Bienne (CH)
(74) Representative: Frei Patent Attorneys

(57) **Abstract**

A distributed system for resource management for hand hygiene units, comprises supervisor units (2) and hand hygiene units (1) with
• a water supply (11a, 11b), water control (12a 12b) and optionally a water metering device (22),
• an additive supply (14), a mixing unit (13) for mixing additive from the additive supply (14) with water, and an additive metering device (23),
• a communication unit (27),
• a data processor (20) configured for
∘ reading data from the additive metering device (23) and optionally the water metering device (22),
∘ determining operating data representing a consumption of consumables, and transmitting the operating data to supervisor units (2), with an identity of the hand hygiene unit (1) being associated with the operating data;
the supervisor units (2) doing at least one of:
• planning of maintenance actions for the hand hygiene units (1);
• monitoring of consumption of consumables.

## Description

The invention relates to the field of hand washing devices and systems. It relates to a distributed system for resource management for hand hygiene units and a distributed system for hand hygiene.

It is known to equip devices located in e.g. washrooms with sensors that measure the consumption of consumables such as air deodorisers, paper towels etc. and report corresponding sensor values to a supervisory system, which then can alert an operator to refill a device.

WO 2015/165731 A1 describes a method for managing a large number of distributed dispensing and related devices by automatically having each device transmit, to a supervising entity, a device data structure that describes properties of the device. This allows for easy integration of each device into the overall system.

Given public sensitivity and regulatory requirements with regard to environmental issues such as the consumption of resources of all kinds (e.g. in international standard ISO 20121 for sustainable event management) there is a need for improvement of current systems.

Guidelines and methods regarding new building conception, building refurbishment and management of buildings, like LEED (Leadership in Energy and Environmental Design) certification or BREEAM (Building Research Establishment Environmental Assessment Method) address resource consumption during construction, refurbishment and operation of buildings. The constructor must be able to consolidate data about installations consumptions and improvements solutions.

It is therefore an object of the invention to create a distributed system for resource management for hand hygiene units and a distributed system for hand hygiene of the type mentioned initially, which addresses at least one of the issues mentioned above.

Another possible object of the invention is to create a distributed system that allows to optimise the effort and costs for operating a plurality of hand hygiene units.

Another possible object of the invention is to create a distributed system that allows to provide data that can be used for modifying facilities and/or planning new facilities with regard to the placement and configuration of hand hygiene units.

Another possible object of the invention is to create a distributed system that allows to provide data required for obtaining sustainability certification.

At least one of these objects is achieved by a distributed system as claimed.

According to a first aspect of the invention, the distributed system for resource management for hand hygiene units comprises
a plurality of hand hygiene units, and one or more supervisor units associated with the plurality of hand hygiene units,
each hand hygiene unit comprising
- a water supply, being either a water tank or a connection to a mains water supply,
- a water control unit, being either a pump or a valve,
- optionally a water metering device for determining an amount of water provided,
- an additive supply, in particular a soap supply,
- a mixing unit for mixing additive from the additive supply with water from the water supply (which then is dispensed to a user),
- an additive metering device for determining an amount of additive provided,
- a communication unit that is configurable to communicate with at least one supervisor unit (by wireless or wire based communication)
- a data processor configured for
   ∘ reading data from the additive metering device and optionally from the water metering device,
   ∘ determining operating data representing a consumption of consumables, the consumables comprising at least the additive and optionally the water, the data optionally also representing a number of usage cycles of the hand hygiene unit,
   ∘ transmitting the operating data thus determined through the communication unit to the one or more supervisor units, with an identity of the hand hygiene unit being associated with the operating data;
   the one or more supervisor units being configured to do, on the basis of the operating data from a plurality of hand hygiene units, at least one of:
- planning of maintenance actions for the plurality of hand hygiene units they are associated with;
- monitoring of consumption of consumables by the plurality of hand hygiene unit or a subset of the plurality of hand hygiene units, and outputting an aggregated consumption, optionally in human readable form.

The term "distributed" means that elements of the system are locally or geographically separated.

The term "hand hygiene unit" is understood to comprise units directed to hand washing, disinfection, application of liquids and other additives for cosmetic purposes. Although the application of the unit to wash or otherwise treat a user's hands is mentioned throughout the text, application to other body parts is possible as well. Depending on the application, an additive can be a solid or liquid soap, detergent, disinfectant, skin care, medical substance etc.

As a result, given on the one hand each hand hygiene unit's capability to control and measure the consumption of consumables, and on the other hand the communication and planning and/or monitoring capabilities of the entire system, an optimal operation of the entire system with regard to resource consumption can be planned and or monitored. Therein resources comprise on the one hand the consumables that are used in the hand hygiene units and on the other hand the effort for maintaining operation of the hand hygiene units that are part of the system, the effort being related to maintenance (refilling, cleaning, ...) and repairing or replacing the hand hygiene units. Usage patterns determined from monitoring can be used to estimate future resource consumption and to plan maintenance actions that lead to better overall efficiency.

The system can allow to implement monitoring of the consumption/usage of consumables that comprise not only an additive such as soap but also water or energy. Furthermore, it allows to optimise its usage and minimise wasting, This becomes more and more important as clean water and drinking water become a scarce resource around the world.

A building constructor and/or operator can consolidate data about use of consumables and resources and determine the effect of improvements to the physical arrangement of hand hygiene units and a maintenance scheme. Every single installation can be assessed in terms or consumption in order to define where to make improvements. Thereby, at least a partial answer to requirements according to the initially mentioned standards and guidelines is provided.

Even more, it allows to minimise facility management and related costs. This can have a significant effect, given that the majority of the cost of a building resides in the operational costs accrued over its lifetime. Such costs can be not only costs of the consumables themselves but also for resources such as work, transporting means and storage locations for storing and distributing the consumables.

Data collected from monitoring allows to make statistics and to build a profile of users of a specific facility (cafeteria, restaurant, male or female restroom, waiting room, etc.) which can be used to tailor a new facility or hand hygiene installation. Statistics gives the trends of consumption within a span of time.

Data collected from monitoring allows to have a lean storage management of consumables stocks, preventing to exceed the expiration date of consumables or limiting the size of storage locations areas within an infrastructure.

Planning of maintenance actions can include optimising facility management by adapting a consumables delivery chain and a next planned service to actual consumption. Thanks to the wealth of data acquired over the distributed system, predictive models can be maintained and the maintenance actions can be planned pro-actively rather than reactively. This in turn allows for more flexible planning, efficient schedules and improved service quality.

Monitoring consumption and communicating consumption to users makes them aware of consumption. Once people are aware of consumption, they can be motivated to act to save resources.

The scope of the system can range from a single washroom or the floor of a building with a set of hand hygiene units, over a building with several such groups of units, to a building complex or an entire city.

The water metering device determines the volume of water being delivered. It can, for example, be implemented with a sensor which directly measures the flow of the water from the speed or the volume of water passing by the sensor, or it can measure the throughput of a pump by counting revolutions of the pump, or it can use a time measuring unit which determines the time during which a valve is open or a pump is activated, and compute the corresponding amount of water from this time.

The additive metering device can operate in a similar manner. In particular, the additive metering device can count the number of turns or an angle of rotation of a pump that delivers the additive, e.g., of a pump.

Associating the identity of the hand hygiene unit with the operating data typically is done explicitly by transmitting a unique identifier that identifies the hand hygiene unit together with the operating data. Alternatively, this association can be established by the fact that a communication link is established between the hand hygiene unit and a supervisor unit with which the hand hygiene unit communicates, so the supervisor unit knows a network address of the hand hygiene unit, and thus a unique identifier of the hand hygiene unit.

In embodiments, at least a subset of the plurality hand hygiene units further comprises a paper dispenser with a paper supply sensor, their data processor being configured for determining, based on information from the paper supply sensor, data representing an amount of paper dispensed by the paper dispenser (which thus also is a consumption of a consumable) and from transmitting this data through the communication unit as part of the operating data.

The paper supply sensor can count turns of a paper roll or a number of paper sheets that are dispensed.

In embodiments, the one or more supervisor units are configured to do the planning of maintenance actions by scheduling when to perform refilling of the consumables for all or a subset of the hand hygiene units, thereby taking into account the physical locations of these hand hygiene units and minimising the effort for the refilling

This effort can be one of or a combination of: distance travelled, time required, energy required, and the number of workers involved. The planning can further take into account factors such as a relative importance of a hand hygiene unit, expected future use (based on historical data), availability of alternative hand hygiene units that are located nearby, in particular in the same room.

Maintenance can, in addition or alternatively to refilling consumables, also involve repairing or replacing a hand hygiene unit or replacing broken parts.

Planning can also involve ordering the delivery of consumables to replenish a local stock of that consumable. Orders for consumables can be transmitted to an ERP system.

The operating data can also comprise diagnostic information about the status of the hand hygiene unit, and error messages in the case of failures. This information can also be used in planning maintenance to check and/or repair a hand hygiene unit.

The hand hygiene unit can comprise further sensors such as an additive level sensor and/or a water level sensor and/or a wastewater level sensor. Information from these sensors can also be part of the operating data and can optionally also be used for the planning and/or the monitoring.

The hand hygiene unit can comprise further sensors providing feedback about the environment of the hand hygiene unit, such as a camera, infrared detectors (signalling the presence of a person), light detectors (reporting level of light, estimating power use by lighting, indicating the need to change a bulb), smell/odor detectors (indicating a need for cleaning), maintenance/cleaning devices or interfaces (registering performed maintenance/cleaning acts, e.g. with digital signatures and checklists of the work done within the environment of a specific unit), user interfaces (allowing a user to register an issue in the washroom and the need of a maintenance intervention).

In embodiments, the communication unit or a further communication unit of the hand hygiene unit is arranged to communicate with further devices located near the hand hygiene unit, to receive information from these further devices and to forward the information to the one or more supervisor units. Optionally the hand hygiene unit is configured to receive information from the one or more supervisor units and to forward the information to the further devices.

This information can comprise data regarding further consumables and/or status data of the further devices. The data can be transmitted to the supervisor units through the communication unit as extended operating data and used for the planning and/or the monitoring. In this way, the communication network established by the hand hygiene units and supervisor units and the planning/maintenance capabilities are extended to cover further devices. Such further devices can be air fresheners or room deodorisers (which need to be refilled), standalone paper towel dispensers, light fixtures (which may report on broken or degraded lights).

In the general sense, the communication infrastructure provided by the hand hygiene units and supervisor units (or only by the supervisor units) can be used to connect a multitude of devices that form part of the "internet of things" (IoT).

The set of supervisor units and optionally also hand hygiene units can form a network in which the unit's communication units provide wired or wireless access points, wired or wireless relays, and Small Cells or Microcells of a cellular (mobile phone / smartwatch) network. Furthermore, a combination of communication standards can be implemented (Bluetooth or NFC for close range, WiFi or Ethernet or cellular standard for longer ranges) and can also incorporate devices of users.

In embodiments, the one or more supervisor units are configured to do the monitoring of consumption of consumables by determining and displaying in human readably form at least one of:
- individual operating data for a hand hygiene unit;
- aggregated operating data for a group of hand hygiene units;
- usage patterns for a hand hygiene unit or for a group of hand hygiene units.

Such usage patterns can comprise an analysis of the distribution of usage of consumables and/or usage cycles over time and/over location of the hand hygiene units. Savings of consumables (such as water, energy, soap and/or paper) and/or of maintenance effort can be computed and compared in relation to other groups or hand hygiene units or in relation to past operating periods.

Monitoring can involve the computation of statistical values from the operating data, such as, the number of visitors within a certain period of time, the type of visitors (male, female, age, ...) the number of visitors combined with time-related environmental characteristics. Such time related information can be found e.g. in the internet, characteristics being for example, events taking place, the weather, water quality, news, etc.

Data obtained by monitoring can be used for comparison with other systems or groups within the system. It can also be used to prove compliance with legal standards or labels for eco-friendliness.

Data obtained by monitoring or by computation may be stored in a centralized or in a distributed database.
Based on the data obtained by monitoring, an operator can plan and effect the spatial relocation of hand hygiene units, for example, from a location where the hand hygiene units are not used to capacity to a location where there is higher demand for them. This can be done in a permanent setting, for example within a building or a set of buildings. It can also be done in a temporary setting, for example at an event location.

In general, if there are more than one supervisor units, then the above planning or monitoring can be effected by these supervisor units in cooperation. For example, some of the supervisor units can merely forward unchanged or aggregated operating data to a higher layer supervisor unit, whereas some of the supervisor units can perform the planning and/or monitoring for a group of hand hygiene units for which it receives operating data. As another example, the location of each hand hygiene unit can be stored in the hand hygiene unit itself and transmitted together with the operating data, or the location can be stored in one of the supervisor units or in a separate database in association with the identity of the hand hygiene unit, and retrieved when required, given the identity. The location can be defined e.g. by geographical coordinates and/or by an address, building number with floor number and room number etc. It can be determined and stored when installing the hand hygiene unit or when planning the unit's installation.

In embodiments, each one of the supervisor units is itself (physically) part of a hand hygiene unit or is a (standalone) controller unit without a hand hygiene unit.

Such as a controller unit can comprise a controller communication unit and a controller data processor, and optionally user interface elements for input from and output to a user, or a short range communication unit for communicating with a hand-held device that serves as user interface. (WiFi, Bluetooth, NFC, ...).

If the supervisor unit is part of a hand hygiene unit, then the data processing required for implementing the functionality of the supervisor unit can be done by the data processor of the hand hygiene unit.

In embodiments, the supervisor units form a hierarchical structure, in which each supervisor unit operates as a parent unit that is either associated with a group of child units, each child unit being either a hand hygiene unit or a supervisor unit.

In the case in which a supervisor unit is part of a hand hygiene unit, then the supervisor unit is, with regard to this hierarchical structure, considered to be a parent of the hand hygiene unit.

In embodiments, a subset of the hand hygiene units are of a first type, and another subset of the hand hygiene units are of a second type, the hand hygiene units of the second type being higher in the hierarchical structure than those of the first type, and those of the second type having at least one of larger processing power and larger transmission rates than those of the first type.

Larger processing power means that an associated data processor can process a larger quantity of data or can process the data faster or both...

Larger transmission rates means, for example, that communication is possible over a longer range and/or at a higher transmission rate.

In embodiments, a parent unit is configured
- to forward the operating data received from its child units to its own parent unit; or
- to aggregate the operating data received from its group of child units and to transmit the aggregated operating data to its own parent unit as group operating data.

Aggregating the operating data can comprise adding the consumption of consumables from the child units. In this case, the consumption is expressed as the amount of the consumable that was consumed over a given time span, such as an hour, a day, a week, etc. This amount can be represented by an absolute value (millilitres, number of tissue sheets, ...). The consumption for each child unit for this time span can be added, giving the total consumption for this group for this time span.

Alternatively, the consumption can be expressed by transmitting a message whenever a predetermined amount of a consumable has been consumed. This amount can be represented by an absolute value (millilitres, number of tissue sheets dispensed, ...) or as a value relative to the capacity for the respective consumable, e.g.% of the capacity of the soap container. In an embodiment, the consumption is only transmitted when the remaining amount of consumable has reached a predetermined lower limit. This can trigger the maintenance planning to schedule a replacement in the near future. It also leads to only few communication events and correspondingly to a low energy expenditure for communication.

The parent/child relationship can be recursive, in that child units themselves can be parent units. In this way a number of groups can be grouped together, forming a group at a higher layer. Correspondingly, operating data from hand hygiene units at a bottom layer can be aggregated in groups on a first intermediate layer, and the aggregated data from these groups can be further aggregated in groups on a second intermediate layer, etc. up to a top layer at which data is aggregated from all hand hygiene units of the system. Likewise, operating data transmitted from child to parent can be forwarded to the parent's parent up to the parent at the top layer. Monitoring and planning can be performed at the top layer but alternatively or in addition also at intermediate layers. For example, statistics generated by monitoring can be established by a supervisor unit for a group at one intermediate layer, e.g. for a building and also by a higher layer supervisor unit for a group at a higher intermediate layer, e.g. for a complex of buildings.

Planning can be realised in different ways: centralised planning wherein a top layer planner takes into account the state and location of all hand hygiene units in the system (say, a building complex or city) and plans the maintenance for each of them. In distributed planning the top layer planner does not know the detailed location of each hand hygiene unit but only the location of a group (building, story, ...) and plans for supply on the layer of such groups. Local planning then takes care for planning the supply of the individual hand hygiene

In embodiments, the operating data comprises operating values that are determined by an input from a user of the hand hygiene unit. Such operating values can be determined by a user choosing or setting a value by means of a user interface, or by the user interacting with the hand hygiene unit in the course of a usage cycle. A user interface can be by means of buttons or a touchscreen but preferably is a contactless input method, e.g. as mentioned below in the context of user feedback. Interacting with the hand hygiene unit can involve withdrawing one's hands from the vicinity of the outlet, activating a proximity switch, a photo sensor, a light barrier, a foot switch, etc.

Such operating values determined by a user can be one or more of:
- a duration of a rinsing phase of each usage cycle, the rinsing phase being terminated by a user withdrawing the hands from the hand hygiene unit or when a maximum rinsing time is reached.
- an amount of soap (or, in general, of an additive) being dispensed.
- for each of the phases of a usage cycle, its duration. Such phases can be, but are not limited to: soap dispensing, intermediate and rinsing phase.

In embodiments, the system is configured to forward display data from one or more supervisor units to one or more hand hygiene units, the hand hygiene units being configured to display the display data on a display of the hand hygiene unit.

In embodiments, the display data is associated with an individual hand hygiene unit or with a group of hand hygiene units and the system is configured to display the display data only on the displays of this individual hand hygiene unit or group of hand hygiene units.

Such a group can be the group of all hand hygiene units that are part of the system, or for example the group of hand hygiene units within entities such as the same city, the same company, the same building, on the same floor or the same room. The display data then typically is tailored for this group. It can comprise information about current events related to the entity corresponding to the group, motivational information, information determined by the monitoring of the group, etc.

The display data can show information that informs the user that the hand hygiene unit is out of one or more consumables and inform her/him about the location of the nearest hand hygiene unit that can be used.

In embodiments, one or more of the hand hygiene units are configured to accept a user input and forward it to the one or more supervisor units.

This allows to display information that prompts a user to perform an input action that generates the user input, for example, pressing a button or an area on a touch screen. For example, the information displayed und the user input can relate to the user satisfaction with the state of the room the hand hygiene unit is located in, or to the washing experience. In this way, user feedback can be collected. If the user feedback relates to the state of the room, e.g. to the cleanliness of a restroom, then this information can be used for the planning of maintenance operations, in particular for the planning of cleaning operations.

For hygienic reasons, user feedback can be through a contactless input method, for example by voice input or gestures or a user touching points on her/his own skin (skin buttons, SkinTrack system) or through an application running on the user's smartwatch or mobile phone, etc.

In embodiments, the system is configured to transmit operating parameters from one or more supervisor units to one or more hand hygiene units, the hand hygiene units being configured to adapt their operation according to the operating parameters.

The hand hygiene unit is typically configured such that a usage cycle begins with a soap dispensing phase, in which a mixture of soap and water is dispensed. The soap dispensing phase (and the entire usage cycle) can be started by a user placing her or his hands under a dispenser, which is detected by a proximity sensor. It is followed by an intermediate phase in which the user can wash her or his hands by rubbing them together. This is followed by a rinsing phase in which only water is dispensed. The rinsing phase can have a fixed maximum time duration. If the user withdraws the hands before this duration, this can be detected and the rinsing can be stopped.

For example, if the hand hygiene unit is known to stand in an environment where relatively intensive handwashing is required, such as a restaurant kitchen or a garage, then the amount of soap to be dispensed in each usage cycle can be increased. The duration of one or more phases of the usage cycle (see below) can be increased as well. Given the knowledge about the environment and the identity of the hand hygiene unit, an operator can set the corresponding operating parameter(s) at one of the supervisor units. The system then transmits the operating parameter(s) to the hand hygiene unit, which then is controlled in according to the transmitted operation parameter(s).

Adapting the operation can be based on external factors like cultural habits, local water hardness, availability of water (drought, water cost), controlling the compliance to standards when a building or location or event is regulated, etc. Thereby, consumption can be controlled and limited according to specific circumstances.

In embodiments, the operating parameters comprise at least one of
- amount of soap per usage;
- soap flow during dispensing phase
- water flow during at least one of a soap dispensing phase, an intermediate phase and a rinsing phase
- time duration parameters for at least one of a soap dispensing phase, an intermediate phase and a rinsing phase.

In embodiments, the system is configured to adjust the operating parameters based on the operating data.

This adjusting can be based on the operating data of one or more hand hygiene units, and/or statistical values derived from the operating data, and/or estimated future operation data representing an expected future consumption.

For example, the operating parameters can be modified to use a little less soap or water (if from a local tank) in case the local supply is running low and a refill is scheduled, but based on past statistics one would expect the supply to be empty by the time the refill will take place and an earlier refill would be costly. Such past statistics can be used to make a prediction of expected usage cycles in the near future, depending, for example, on the weekday, weather, occurrence of certain events, etc. This modification of operating parameters can optimise the dispensing of consumables, taking into account a cost for an early maintenance operation for refilling, optionally satisfying constraints such as a minimum amount of consumables per usage cycle.

Such a modification of the operating parameters can be performed by a higher or top layer supervisor unit. On a local or lower layer, if within a group of hand hygiene units a consumable is running low, the hand hygiene units can be locally coordinated, for example by a unit on the first intermediate layer, to do one or more of:
- reduce their consumption of consumables per usage cycle;
- indicate that it is not in operation and indicating to a user the location of the nearest hand hygiene unit that is in operation.

In embodiments, the system is configured to determine a type of soap used in a particular hand hygiene unit and to adapt at least one operating parameter of the hand hygiene unit according to the type of soap.

For example, if a particular type of soap requires a larger volume of soap to be dispensed for a satisfactory washing effect, a predetermined volume to be dispensed in the soap dispensing phase is increased by setting a corresponding operating parameter of the hand hygiene unit.

Similarly, operating parameters can be adapted in accordance with water hardness and/or regulatory requirements.

Determining the type of soap can be done by automatically (reading a code on the soap container) or manually identifying the type of soap when a soap container is replaced or refilled in the hand hygiene unit, or by, when scheduling the hand hygiene unit for being serviced, storing the type of soap to be used.

Here too, the association between the type of soap and the operating parameters, and the steps for determining, storing, and passing down operating parameters to the hand hygiene units can be performed at different layers of the hierarchy of supervisor units. A database comprising information about different types of soap and information on how to determine the operating parameters depending on the type of soap and/or water hardness can reside in one of the supervisor units or as a separate unit that can be accessed by the supervisor units.

According to a second aspect of the invention, a distributed system for hand hygiene is provided, comprising
- a plurality of hand hygiene units, and one or more supervisor units associated with the plurality of hand hygiene units;
- each hand hygiene unit comprising metering means for collecting operating data,
- the system implementing a distributed computational structure with at least a lower layer and a higher layer;
- the lower layer comprising data processors and communication units of a plurality of hand hygiene units, the data processors at the lower layer being configured to determine operating data and location reference data for the respective hand hygiene unit and to transmit this operating data and location reference data to the higher layer; the data processors at the lower layer *optionally* being configured to adapt the operation of the respective hand hygiene unit according to operating parameters;
- the higher layer comprising data processors' and communication units' of one or more supervisor units, the data processors' at the higher layer being configured to do, on the basis of the operating data from a plurality of hand hygiene units, *at least one of:*
   ∘ planning of maintenance actions for the plurality of hand hygiene units they are associated with;
   ∘ monitoring of consumption of consumables by the plurality of hand hygiene unit or a subset of the plurality of hand hygiene units, and optionally outputting an aggregated consumption in human readable form.

The hand hygiene unit in the second aspect of the invention can be of the same kind as that described in the context of the first aspect of the invention, in particular comprising a water supply, control unit and metering device, an additive supply, mixing unit and additive metering device, and a communication unit. Alternatively, the hand hygiene unit can comprise only an additive supply and additive metering (and thus no water supply and mixing unit). In this case the additive is the only substance delivered and can be simply be called a "substance".

The location reference data can be location data specifying the location of the hand hygiene unit, or an identifier of the hand hygiene unit that allows to retrieve its location data, typically from a database that is maintained at another layer.

In embodiments, the system comprises an intermediate layer, the intermediate layer comprising data processors and communication units, of a plurality of hand hygiene units and supervisor units, these data processors being configured
- to forward operating data received from the lower layer to the higher layer; or
- to aggregate operating data received from the lower layer and to transmit the aggregated operating data to the higher layer;
- and, optionally, to forward operating parameters received from the higher layer to the lower layer.

In embodiments, the system comprises distributed generic communication software units being executed on each of the data processors of the hand hygiene units and supervisor units, the communication software units in collaboration implementing a communication structure of the system, by
each communication software unit having an identifier, and being parameterised or configured by
- having the identifiers of zero, one or more children associated with it and by
- having the identifier of zero or one parents associated with it, and
- by being configured, if it has one parent associated with it,
   ∘ either to forward information received from a child, including the child's identifier, to its parent,
   ∘ or to aggregate information received from two or more of its children and transmit the aggregated information, including its own identifier, to its parent;
- by being configured, if it has one or more children associated with it, to forward information received from its parent, to one or more of the children.

In order to let a parent at a higher layer know via which unit at an intermediate layer a particular child can be reached, each parent can transmit the identifier of its children, in association with its own identifier, to its parent. This information can be stored in the receiving parent and forwarded upwards to the parent at the next higher layer. This is repeated up to the top layer parent. As a result, each unit, given the identifier of a lower layer unit that is directly or indirectly under it, has the information to determine through which intermediate units information can be sent to the lower layer unit.

Further embodiments are evident from the dependent patent claims.

The subject matter of the invention will be explained in more detail in the following text with reference to exemplary embodiments which are illustrated in the attached drawings, in which:
- Figure 1: schematically shows a hand hygiene unit; and
- Figure 2: shows a structure of a distributed system for resource management for hand hygiene units.

In principle, identical parts or parts that are functionally similar are provided with the same reference symbols in the figures.

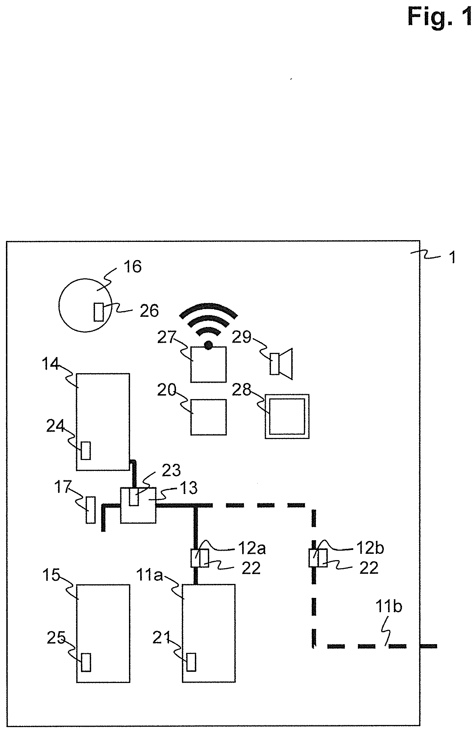

**Figure 1** schematically shows a hand hygiene unit 1. It comprises a water tank 11 a and a liquid supply 14 for supplying an additive in the form of a liquid, typically soap or a detergent or cosmetic preparation. The liquid supply 14 can be equipped with a liquid detection or level sensor 24. As an alternative or in addition to the water tank 11a, a mains water supply 11b can be present. The flow of water from the water tank 11 a is controlled by a pump 12a. The water tank 11a can be equipped with a water detection or level sensor 21. The flow of water from the mains water supply 11b is controlled by a valve 12b, preferably in combination with a flow regulator. The flow regulator ensures that the flow rate is - within limits of course - largely independent of a mains pressure. In both cases, the flow can be measured with a water metering device 22. Depending on the type of water supply, the water metering device 22 can be a flow meter, a device for registering an angle or number of turns of a pump (a counter of pump work), or a device for determining the opening time that a valve is open, which for a given constant water flow allows to determine the amount of water passing through the valve. If the valve is controlled by the hand hygiene unit 1 itself, then the water metering device 22 need not be a physical sensor but can be realized by a controller that determines the opening time. The water and the liquid are combined by a mixing unit 13 and dispensed through an outlet to a user for hand washing. The flow of liquid is determined and/or measured with a liquid metering device 23. The mixing unit 13 can also add air to the mixture of water and liquid before the dispensing, which can lead to a good washing experience while consuming relatively little amount of water and soap. A presence detector 17 is arranged to detect when a user's hands are placed under or near the outlet.

The liquid supply 14 alone, or the liquid supply 14 together with the mixing unit 13, can be a replaceable component which plugs into a base unit of the hand hygiene unit 1. When the liquid supply 14 is empty, the complete component can be replaced rather than filling the liquid supply 14.

After dispensing, the used water/liquid mixture can be collected in a wash basin and be led down a drain (not shown) or be collected in a wastewater tank 15, which can be equipped with a wastewater level or wastewater detection sensor 25.

Optionally, a paper dispenser 16 can be provided, which can be equipped with a paper supply sensor 26.

A dispensing unit comprises the physical devices delivering and/or mixing the substances and liquids. In different exemplary embodiments, the dispensing unit can comprise
- just an additive or liquid supply 14 and an additive or liquid metering device 23.
- the above, and additionally a mixing unit 13 and a water supply and water control unit.
- the above, and additionally a wastewater tank 15.

In each case, such a dispensing unit can be associated with and controlled by a dedicated data processor 20, or two or more dispensing units can be associated with and controlled by a single, common data processor 20. A dispensing unit can be realized with all the respective elements, optionally also a data processor 20, in a single, stand-alone housing. Alternatively, some elements can be incorporated in a faucet, with the remaining elements in a separate housing near the faucet.

In an embodiment, at least a mixing unit 13, a liquid metering device 23 and a water valve 12b are incorporated in a faucet that is designed to be mounted to a wall or to a washbasin. An additive or liquid supply liquid supply 14 can also be incorporated in the faucet. The data processor 20 can be incorporated in the faucet or in a separate enclosure.

Operation of the hand hygiene unit 1 is controlled by a controller in the form of a data processor 20 arranged to read sensor values from the different sensors and to control the pump 12a and/or valve 12b. In particular, the data processor 20 is arranged to initiate a usage cycle when a user's hands are detected by the presence detector 17. A usage cycle begins with a soap dispensing phase, optionally followed by an intermediate phase for rubbing the hands together or scrubbing, and then a rinsing phase in which only water is dispensed. Depending on which phase the unit is in, the valve 12b or pump 12a and the mixing unit 13 are controlled by the data processor 20 to produce and dispense the desired mixture. As a rule, the duration of each phase is not controlled by the user but is predetermined. Only the rinsing phase can be cut short by the user withdrawing his or her hands.

In embodiments (not illustrated), a data processor 20 is arranged to control more than one units comprising a mixing unit 13, liquid supply 14 wash basin etc.

The data processor 20 is arranged to communicate with other hand hygiene units 1 or with supervisor units 2 through a communication unit 27. This typically is a wireless unit, using e.g. a WiFi, Cellular (GSM, UMTS, LTE etc.), Bluetooth or other standard. Alternatively, or in addition, it can use a wire-based standard such as Ethernet or a powerline communication system. The data processor 20 can be arranged to drive user interface elements such as a (touchscreen) display 28, a loudspeaker 29 and input elements such as buttons or contactless input elements. Contactless input elements or methods can be the following, as alternatives to touching the screen:
- capacitive sensors/elements;
- optical sensors, especially infrared sensors, visual light sensors, laser based sensors, e.g., sensors based on photoelectric, photoconductive, photovoltaic photoelectrochemical effects (examples: proximity sensors, light barriers);
- ultrasonic sensors, e.g., distance sensors, sonar/doppler-effect based sensors;
- inductive sensors;
- Hall sensors or Reed switches, or other sensors based on electric or magnetic fields, e.g., based on "Radar" or on eddy currents, or magneto- or electro-resistive or -conductive sensors, or magneto- or electro-chemical sensors;
- acoustic sensors, e.g., microphones, or acoustic near field sensors;
- voice commands;
- sensors included within a watch or ring;
- sensors combined to hand orientation;
- hand gesture combined with wearables;
- buttons projected on the user's skin;
- skintrack sensors (normally used to enlarge screen of smartwatches);
- communicating with a user's smartwatch or smartphone.

An example of a mixing unit 13 with a liquid metering device 23 that can be used in a hand hygiene unit 1 is described in further detail in WO 2006/005401 A2.

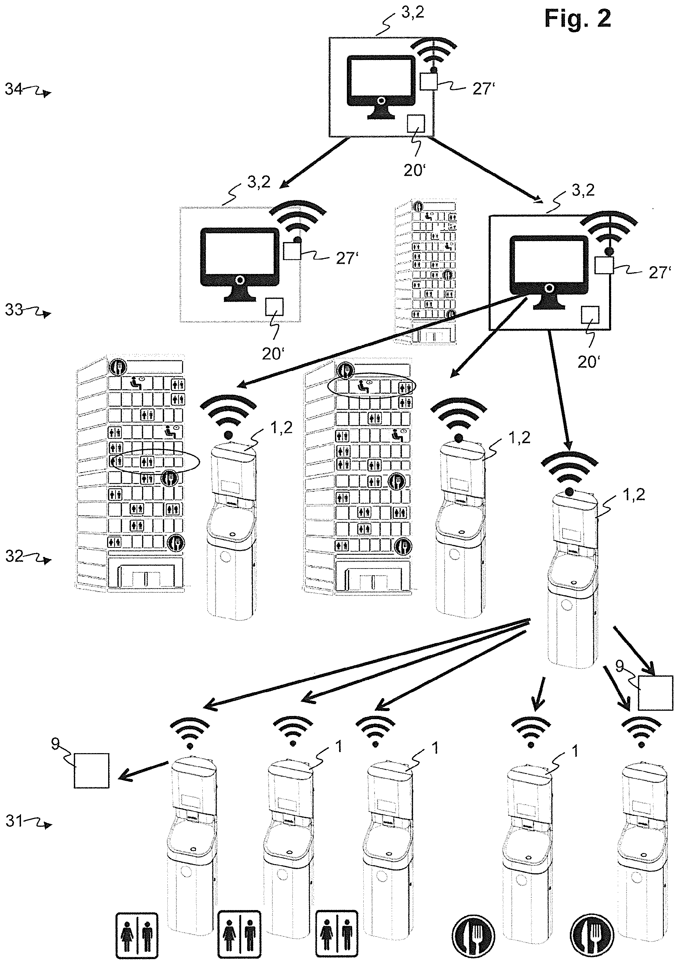

**Figure 2** shows a hierarchical structure of a distributed system for resource management for hand hygiene units 1. At a lowest layer or bottom layer 31 are hand hygiene units 1 which in this example can be desktop units supplied by a mains water supply and provided with a drain, or can be standalone units comprising a water tank and a wastewater tank. The hand hygiene units 1 can be located in restrooms, kitchens, waiting rooms etc. or be temporarily deployed to an event location.

At a first intermediate layer 32 are parent hand hygiene units 1 that are configured to communicate with the hand hygiene units 1 at the bottom layer 31. Together, they form groups that are associated with and distributed over, for example, one floor or adjacent floors of a building. The figure shows, on the first intermediate layer 32, three such groups, with the corresponding hand hygiene units 1 on the bottom layer 31 drawn only for the rightmost group. Communication between the members of the group should cover distances in the range of tens of meters but generally less than a hundred meters. In each group, one of the hand hygiene units 1 serves as parent unit for the child units at the bottom layer 31.

At a further higher layer, here a second intermediate layer 33, groups of groups are present, with a supervisor unit 2 serving as parent to the parents in the first intermediate layer 32. The supervisor units 2 also comprise data processors 20' and communication units 27' but no further hardware for realising a hand hygiene unit. Each supervisor unit 2 is shown to cover the hand hygiene units 1 of an entire building. The types of data processors 20' of the supervisor units 2 can differ from those of the hand hygiene units 1, or be the same.

At a top layer 34, a top supervisor unit 2 serves as parent to the parents in the second intermediate layer 33.

It is understood that one or more of the hand hygiene units 1 on the first intermediate layer 32 can be replaced with regard to communication and computational tasks by supervisor units 2. Similarly, supervisor units 2 on the second intermediate layer 33 and higher layers can be replaced by hand hygiene units 1 with sufficiently powerful computing and communication powers. As a rule, requirements on communication distance and/or computing power increase as one goes up the layers.

The data processors and communication units of the hand hygiene units 1 and supervisor units 2 are configured to propagate operating data measured by the hand hygiene units 1 sensors upward. Such operating data from a hand hygiene unit 1 is associated with an identity of the hand hygiene unit 1. At each layer in the hierarchy, operating data can either be forwarded, together with the associated identity, up to the next layer, or it can be aggregated at one or more layers, with only the aggregated operating data being transmitted upward. For example, up to the layer of a building complex, the individual operating data for each hand hygiene unit 1 can be propagated upwards, in order to allow for planning maintenance actions in this building complex. Then only aggregated data is transmitted to higher layers, for example a company or city layer, since at that layer no fine grained planning is required, or only data for monitoring is required, e.g. for comparison with other entities or for checking compliance with regulations.

**Figure 2** also shows further devices 9 that are different from hand hygiene units but are arranged to communicate with hand hygiene units 1 or supervisor units 2 at any of the layers. Operating data from such further devices 9 can be processed and used for planning and monitoring in the same manner as the operating data from the hand hygiene units 1. The network of hand hygiene units 1 and supervisor units 2 serves to integrate these otherwise isolated further devices 9 into an extended and integrated system for resource management.

Given information about the level of consumables or just the need to refill one or more consumables, and possibly also about malfunctions or the need for services other than refilling, at each of the distributed hand hygiene units, together with information on the physical or geographical distribution of the hand hygiene units allows to schedule maintenance operations using known scheduling algorithms.

Given information from monitoring, usage patterns determined from monitoring can be used to estimate future resource consumption and to plan maintenance actions that lead to better overall efficiency. For example, assume that a unit is installed in a male restroom and a second one in an adjacent female restroom, and that monitoring shows that the unit installed in the female restroom is used twice as much than the one in the male restroom. Then the planned maintenance actions of the more frequently used unit can be coordinated to coincide every second time maintenance of other unit. For a whole system comprising several hand hygiene units, a maintenance and/or refill schedule can be optimized, for example, such that the person doing the work has the same workload (or time required) for a particular maintenance tour performed at a maintenance interval. Depending on the type of facility, the maintenance interval can be in the range of daily to several days to several weeks. A further use of operating data, in the above example, is to add hand hygiene units in places where a large number of usage cycles occur.

While the invention has been described in present embodiments, it is distinctly understood that the invention is not limited thereto, but may be otherwise variously embodied and practised within the scope of the claims.

## Claims

1. A distributed system for resource management for hand hygiene units, comprising
a plurality of hand hygiene units (1), and one or more supervisor units (2) associated with the plurality of hand hygiene units (1),
each hand hygiene unit (1) comprising
• a water supply, being either a water tank (11 a) or a connection to a mains water supply (11b),
• a water control unit, being either a pump (12a) or a valve (12b),
• optionally a water metering device (22) for determining an amount of water provided,
• an additive supply (14), in particular a soap supply,
• a mixing unit (13) for mixing additive from the additive supply (14) with water from the water supply,
• an additive metering device (23) for determining an amount of additive provided,
• a communication unit (27) that is configurable to communicate with at least one supervisor unit (2),
• a data processor (20) configured for
o reading data from the additive metering device (23), and optionally from the water metering device (22),
∘ determining operating data representing a consumption of consumables, the consumables comprising at least the additive and optionally the water, the data optionally also representing a number of usage cycles of the hand hygiene unit (1),
∘ transmitting the operating data thus determined through the communication unit (27) to the one or more supervisor units (2), with an identity of the hand hygiene unit (1) being associated with the operating data;
the one or more supervisor units (2) being configured to do, on the basis of the operating data from a plurality of hand hygiene units (1), at least one of:
• planning of maintenance actions for the plurality of hand hygiene units (1) they are associated with;
• monitoring of consumption of consumables by the plurality of hand hygiene unit (1) or a subset of the plurality of hand hygiene units (1), and outputting an aggregated consumption, optionally in human readable form.

2. The system of claim 1, wherein at least a subset of the plurality hand hygiene units (1) further comprising a paper dispenser (16) with a paper supply sensor (26), their data processor (20) being configured for determining, based on information from the paper supply sensor (26), data representing an amount of paper dispensed by the paper dispenser (16) and from transmitting this data through the communication unit (27) as part of the operating data.

3. The system of claim 1 or claim 2, wherein the one or more supervisor units (2) are configured to do the planning of maintenance actions by scheduling when to perform refilling of the consumables for all or a subset of the hand hygiene units (1), thereby taking into account the physical locations of these hand hygiene units (1) and minimising the effort for the refilling

4. The system of one of the preceding claims, wherein the communication unit (27) or a further communication unit of the hand hygiene unit (1) is arranged to communicate with further devices (9) located near the hand hygiene unit (1), to receive information from these further devices and to forward the information to the one or more supervisor units (2), and optionally to receive information from the one or more supervisor units (2) and to forward the information to the further devices (9).

5. The system of one of the preceding claims, wherein the one or more supervisor units (2) are configured to do the monitoring of consumption of consumables by determining and displaying in human readably form at least one of:
• individual operating data for a hand hygiene unit (1);
• aggregated operating data for a group of hand hygiene units (1);
• usage patterns for a hand hygiene unit (1) or for a group of hand hygiene units (1).

6. The system of one of the preceding claims, wherein the operating data comprises operating values that are determined by an input from a user of the hand hygiene unit, for example, a duration of a rinsing phase of each usage cycle, the rinsing phase being terminated by a user withdrawing the hands from the hand hygiene unit (1) or when a maximum rinsing time is reached.

7. The system of one of the preceding claims, wherein the system is configured to forward display data from one or more supervisor units (2) to one or more hand hygiene units (1), the hand hygiene units (1) being configured to display the display data on a display (28) of the hand hygiene unit (1).

8. The system of claim 7, wherein the display data is associated with an individual hand hygiene unit (1) or with a group of hand hygiene units (1) and the system is configured to display the display data only on the displays (28) of this individual hand hygiene unit (1) or group of hand hygiene units (1).

9. The system of one of the preceding claims, wherein one or more of the hand hygiene units (1) are configured to accept a user input and forward it to the one or more supervisor units (2).

10. The system of one of the preceding claims, wherein the system is configured to transmit operating parameters from one or more supervisor units (2) to one or more hand hygiene units (1), the hand hygiene units (1) being configured to adapt their operation according to the operating parameters.

11. The system of claim 10, wherein the operating parameters comprise at least one of
• amount of additive per usage cycle;
• additive flow during dispensing phase;
• water flow during at least one of an additive dispensing phase, an intermediate phase and a rinsing phase;
• time duration parameters for at least one of an additive dispensing phase, an intermediate phase and a rinsing phase.

12. The system of claim 10 or 11, wherein the system is configured to adjust the operating parameters based on the operating data.

13. The system of one of the preceding claims, wherein the system is configured to determine a type of additive used in a particular hand hygiene unit (1) and to adapt at least one operating parameter of the hand hygiene unit (1) according to the type of additive.

14. The system of one of the preceding claims, wherein the supervisor units (2) form a hierarchical structure, in which each supervisor unit (2) operates as a parent unit that is either associated with a group of child units, each child unit being either a hand hygiene unit (1) or a supervisor unit (2).

15. The system of claim 14, wherein a parent unit is configured
• to forward the operating data received from its child units to its own parent unit; or
• to aggregate the operating data received from its group of child units and to transmit the aggregated operating data to its own parent unit as group operating data.

16. The system of one of claims 14 to 15, wherein a subset of the hand hygiene units (1) are of a first type, and another subset of the hand hygiene units (1) are of a second type, the hand hygiene units (1) of the second type being higher in the hierarchical structure than those of the first type, and those of the second type having at least one of larger processing power and larger transmission rates than those of the first type.

17. The system of one of the preceding claims, wherein each one of the supervisor units (2) is itself part of a hand hygiene unit (1) or is a controller unit (3) without a hand hygiene unit (1).

18. A distributed system for hand hygiene, comprising
• a plurality of hand hygiene units (1), and one or more supervisor units (2) associated with the plurality of hand hygiene units (1);
• each hand hygiene unit (1) comprising metering means for collecting operating data,
• each hand hygiene unit (1) preferably being as defined in claim 1;
• the system implementing a distributed computational structure with at least a lower layer and a higher layer;
• the lower layer comprising data processors (20) and communication units (27) of a plurality of hand hygiene units (1), the data processors (20) at the lower layer being configured to determine operating data and location reference data for the respective hand hygiene unit (1) and to transmit this operating data and location reference data to the higher layer; the data processors (20) at the lower layer *optionally* being configured to adapt the operation of the respective hand hygiene unit (1) according to operating parameters;
• the higher layer comprising data processors (20') and communication units (27') of one or more supervisor units (2), the data processors (20') at the higher layer being configured to do, on the basis of the operating data from a plurality of hand hygiene units (1), *at least one of*:
• planning of maintenance actions for the plurality of hand hygiene units (1) they are associated with;
• monitoring of consumption of consumables by the plurality of hand hygiene unit (1) or a subset of the plurality of hand hygiene units (1), and optionally outputting an aggregated consumption in human readable form.

19. The system of claim 18, comprising an intermediate layer, the intermediate layer comprising data processors (20, 20') and communication units (27, 27') of a plurality of hand hygiene units (1) and supervisor units (2), these data processors being configured
• to forward operating data received from the lower layer to the higher layer; or
• to aggregate operating data received from the lower layer and to transmit the aggregated operating data to the higher layer;
• and, optionally, to forward operating parameters received from the higher layer to the lower layer.

20. The system of claim 18 or 19, comprising distributed generic communication software units being executed on each of the data processors (20, 20') of the hand hygiene units (1) and supervisor units (2), the communication software units in collaboration implementing a communication structure of the system, by each communication software unit having an identifier, and being parameterised by
• having the identifiers of zero, one or more children associated with it and by
• having the identifier of zero or one parents associated with it, and
• by being configured, if it has one parent associated with it,
∘ either to forward information received from a child, including the child's identifier, to its parent,
∘ or to aggregate information received from two or more of its children and transmit the aggregated information, including its own identifier, to its parent;
• by being configured, if it has one or more children associated with it, to forward information received from its parent, to one or more of the children.

21. The system of one of claims 18 to 20, wherein the one or more supervisor units (2) are configured to do the planning of maintenance actions by scheduling when to perform refilling of the consumables for all or a subset of the hand hygiene units (1), thereby taking into account the physical locations of these hand hygiene units (1) and minimising the effort for the refilling

22. The system of one of claims 18 to 21, wherein the one or more supervisor units (2) are configured to do the monitoring of consumption of consumables by determining and displaying in human readably form at least one of:
• individual operating data for a hand hygiene unit (1);
• aggregated operating data for a group of hand hygiene units (1);
• usage patterns for a hand hygiene unit (1) or for a group of hand hygiene units (1).

23. The system of one of claims 18 to 22, wherein each one of the supervisor units (2) is itself part of a hand hygiene unit (1) or is a controller unit (3) without a hand hygiene unit (1).

24. The system of one of claims 18 to 23, wherein the operating data comprises a duration of a rinsing phase of each usage cycle, the rinsing phase being terminated by a user withdrawing the hands from the hand hygiene unit (1) or when a maximum rinsing time is reached.

25. The system of one of claims 18 to 24, wherein the system is configured to forward display data from one or more supervisor units (2) to one or more hand hygiene units (1), the hand hygiene units (1) being configured to display the display data on a display (28) of the hand hygiene unit (1).

26. The system of one of claims 18 to 25, wherein the display data is associated with an individual hand hygiene unit (1) or with a group of hand hygiene units (1) and the system is configured to display the display data only on the displays (28) of this individual hand hygiene unit (1) or group of hand hygiene units (1).

27. The system of one of claims 18 to 26, wherein one or more of the hand hygiene units (1) are configured to accept a user input and forward it to the one or more supervisor units (2).

28. The system of one of claims 18 to 27, wherein the system is configured to transmit operating parameters from one or more supervisor units (2) to one or more hand hygiene units (1), the hand hygiene units (1) being configured to adapt their operation according to the operating parameters.

29. The system of claim 28, wherein the operating parameters comprise at least one of
• amount of additive per usage cycle;
• additive flow during dispensing phase;
• water flow during at least one of an additive dispensing phase, an intermediate phase and a rinsing phase;
• time duration parameters for at least one of an additive dispensing phase, an intermediate phase and a rinsing phase.

30. The system of claim 28 or 29, wherein the system is configured to adjust the operating parameters based on the operating data.
